(19) Europäisches Patentamt
European Patent Office
Office européen des brevets

(11) EP 3 069 656 B1

## (12) EUROPEAN PATENT SPECIFICATION

(45) Date of publication and mention
of the grant of the patent:
**04.10.2017 Bulletin 2017/40**

(51) Int Cl.:
**A61B 5/0488** (2006.01)     **A61B 5/11** (2006.01)

(21) Application number: **16163864.8**

(22) Date of filing: **27.03.2013**

(54) **SYSTEM FOR THE ACQUISITION AND ANALYSIS OF MUSCLE ACTIVITY AND OPERATION METHOD THEREOF**

SYSTEM ZUR ERFASSUNG UND ANALYSE DER MUSKELAKTIVITÄT UND BETRIEBSVERFAHREN DAFÜR

SYSTÈME POUR L'ACQUISITION ET L'ANALYSE DE L'ACTIVITÉ MUSCULAIRE ET SON PROCÉDÉ DE FONCTIONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2012 IT MI20120494**

(43) Date of publication of application:
**21.09.2016 Bulletin 2016/38**

(62) Document number(s) of the earlier application(s) in accordance with Art. 76 EPC:
**13723229.4 / 2 830 494**

(73) Proprietor: **B10NIX S.r.l.**
**20131 Milano (IT)**

(72) Inventors:
• **MAURI, Alessandro Maria**
  **20146 Milano (IT)**
• **MUTTI, Flavio**
  **29121 Piacenza (IT)**
• **BELLUCO, Paolo**
  **20133 Milano (IT)**

(74) Representative: **Faggioni, Carlo Maria et al**
**Fumero S.r.l.**
**Pettenkoferstrasse 20/22**
**80336 München (DE)**

(56) References cited:
**WO-A1-2006/082565       WO-A1-2010/065067**
**WO-A2-2005/086574       US-A1- 2011 224 530**

**Description**

Field of the invention

The present invention relates to a system for the acquisition an analysis of muscle activity and to a operation method thereof.

Background Art

The need exists in the sports or medical field to perform detecting and recording on an individual's muscle function. An instrument often used for such purpose consists of an electromyographic sensor capable of sampling the electric signal generated by the muscle during the activity of the same. The information which may be obtained by an electromyographic sensor is per se suitable to understand the function and local reactivity of the muscle, but is often insufficient for a full characterisation of the individual, meant as a complex human being, provided with a plurality of muscles subject to a plurality of nervous stimuli and to diverse outer forces (external reactions, but also inertia forces deriving from the dynamics of the individual himself/herself).

Image sensors for acquiring the position of an individual's limbs or of the bone arrangement are also known. This technique is used to be able to control operating devices - for example in the field of videogames - or to operate therapeutically on the human body (possibly with the aid of markers which increase detection accuracy).

Some known devices employing electromyographic sensors or image sensors for the above-said purposes are described for example in WO2010/104879, EP310901 and WO2010/95636.

It has also been suggested to somehow combine the two types of information acquired by electromyographic sensors and by image sensors. Prior art examples are described in JP2009273551 and US2012/4579. However, in these cases the two heterogeneous signals coming from the different types of sensors are processed separately, which does not supply an automatic result directly usable by the non-expert user. US2011/0224530 discloses a system for the acquisition of an individual's muscle activity according to the preamble of claim 1. Other relevant prior art include WO2005/086574, WO2010/065067, WO2006/082565.

Summary of the Invention

The object of the present invention is hence to provide an apparatus and a relative method which overcomes the drawbacks of the prior art, so as to automatically supply information on an individual's muscle activity which is complete, reliable and directly usable by a non-expert user.

Such object is achieved through the features described in the independent apparatus claim 1 and independent method claim 2. The dependent claims describe preferential features of the invention. Furthermore, a system is disclosed which comprises two distinct acquisition sections and a processing unit capable of recognising the actions performed by a user, both from a kinematic and from a dynamic point of view. The system is hence capable of understanding the user's actions by utilising a system of videocameras and of electromyographic sensors applied to the user's body, aided by other biometric support sensors, preferably of a type and amount sufficient to develop also processing techniques for the understanding of the user's emotional state (affective computing).

Through the use of videocameras (first acquisition section), the system is capable of acquiring position, velocity and acceleration features of the user's various body parts; while through the electromyographic technique (aided by other biometric sensors) information on the same user's effort, fatigue and muscle tension is acquired.

The system is hence capable of recognising in real time all these signals, of merging them, through artificial intelligence techniques, to extract the information on the user's action and to actively interact with the user through the knowledge and understanding of this information. In particular, the system is capable of measuring the muscle activity and the kinematics of the user's movement, it is hence capable of determining the amount of activation of the various muscle groups (firing rate, fatigue, width and velocity of activation of the motor units) together with the user's movement (position, velocity and accelerations of the user's body parts), in order to have a full measurement of a given action in various different fields, for example in the sports field, rehabilitation and game field.

Brief Description of the Drawings

Further features and advantages of the invention are in any case more evident from the following detailed description of preferred embodiments, given purely as a nonlimiting example and illustrated in the attached drawings, wherein:

fig. 1A is a general flow chart of the system according to the present disclosure;

fig. 1B is a schematic view of an acquisition and analysis system according to the present disclosure;

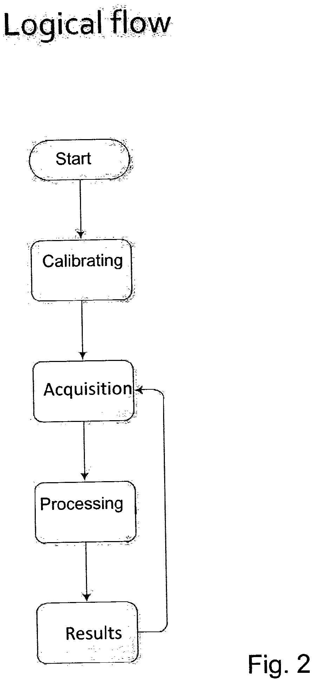

fig. 2 is a flow chart of the operation process which takes place in the system according to the present disclosure;
fig. 3 is a flow chart of the first calibration phase which occurs in the system according to the present disclosure;
fig. 4 is a flow chart of the second acquisition phase which occurs in the system according to the present disclosure;
fig. 5 is a flow chart of the third processing phase which occurs in the system according to the present disclosure;
fig. 6 represents a series of exemplifying diagrams of some electromyographic and video-time-based signals acquired depending on the time by the system according to the invention;
fig. 7 is an exemplifying flow chart of the processing of the electromyographic signal;
fig. 8 is an exemplifying flow chart of the processing of the video signal; and
fig. 9 is an exemplifying drawing showing a possible application of the system according to the invention.

Detailed Description of Preferred Embodiments

With reference to the drawings, an acquisition and analysis system of an individual's motor activity consists of two main signal acquisition sections: a first section A for the acquisition of electromyographic signals and a second section B for the acquisition of video signals. In substance, on the one hand, a video device acquires the moving image of a user, supplying a corresponding video signal; on the other hand, a plurality of electrodes of an electromyographic device are applied in preset positions of the user's body, acquiring electromyographic signals (i.e. signals of the electric activity in the muscles) through an EMG card.

The signals acquired in the two sections A and B are processed and mutually coordinated to supply an output signal which is not given by the simple sum of the two signals, but that is the result of a correlation of the two signals one on the other and which hence supplies a synergistic outcome with respect to the one obtainable from the sum of the signals acquired in the two sections.

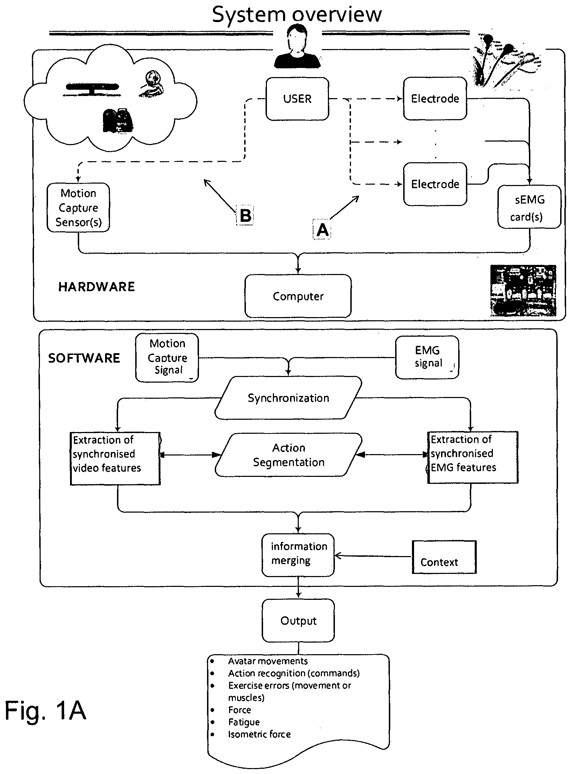

In the lower part of fig. 1A the processing of the two signals is briefly represented. The video signal 'motion capture' and the electromyographic signal 'EMG' are acquired and synchronised in time. Subsequently it is provided to extract the synchronised video features and the synchronised EMG features and to superimpose them on a plurality of frames into which a dynamic action of the individual's body is split. Subsequently the synchronised video features and the synchronised EMG features, split on the actions, are merged together, using a corrective or ponderal factor which takes the context into account: in such sense, the supplied measurement is of a parametric type.

The process is thus capable of supplying an output which is determined by the two original signals deriving from the two distinct acquisition sections, and which is furthermore influenced by the context and by the mutual influence between the video signals and EMG signals. The output signal can be used to control the movements of an avatar (for example on videogame consoles), for a generic calibrated recognition of the individual's actions (for example for the performing of specific orders), for the detection of errors in the individual's modes of movement (for example for the purpose of a corrective action on a sports exercise or on the application of a force) and so on.

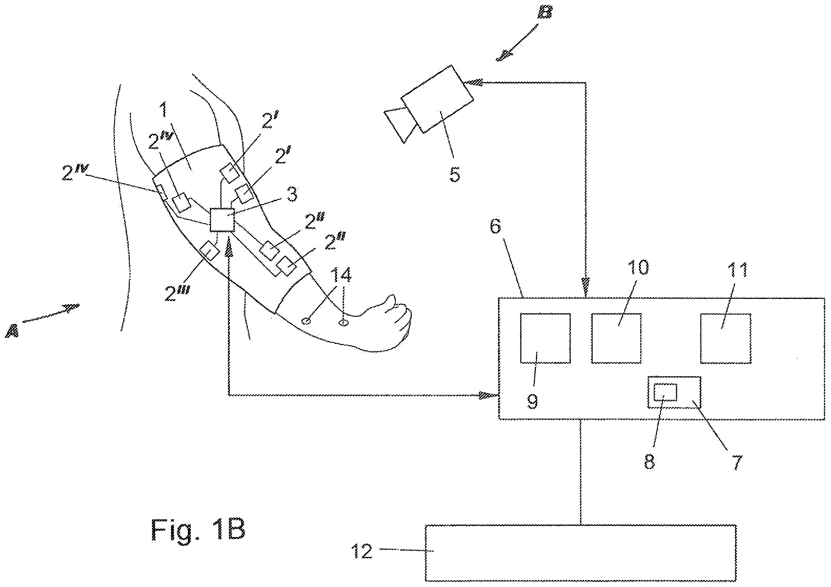

Fig. 1B schematically shows a possible configuration of the hardware.

According to the invention, to the first acquisition section A belongs a garment 1 (band, sweater, wetsuit, trousers, etc...) provided with electrodes 2 and with a surface electromyographic acquisition card (SEMG) 3 with one or preferably more channels for each electrode 2 or pair of electrodes 2, capable of acquiring, filtering and sampling the electric signal issued during a muscle contraction; with reference to an arm activity, an advantageous configuration suitable for the system of the invention implies to have a pair of $2^i$ electrodes on the biceps, a pair of electrodes $2^{ii}$ on the brachio-radial muscle, a reference electrode $2^{iii}$ on the elbow and a pair of electrodes $2^{iv}$ on the triceps.

To second acquisition section B belongs a video sensor (RGB camera, depth-map camera, IR motion capture camera with marker, motion sensing input device) 5, capable of acquiring a sequence of images (at a preset frequency, for example of 30 Hz) of the individual's movement dynamics or of a specific part of his/her body (in the illustrated example, the arm movement).

The system furthermore consists of a processing unit 6 to which the electromyographic card 3 and video sensor 5 are connected in any known way, for example by cable or in a wireless manner (Radio Frequency, InfraRed, etc...).

The computer unit or processor 6 in turn has a signal synchronisation device 7, provided with means 8 for the generation of a single reference time signal (reference clock) for the acquisition of signals from electromyographic sensor 3 and from video sensor 5. It is hence provided to synchronise the acquisition of the two signals, through a time marker (timestamp) supplied by the reference clock, which is assigned to each data acquisition by each sensor. Computer processor 6 furthermore has means 9 for the analysis and disjoint processing (i.e. filtering and cleaning of each signal based on the type of sensor) and/or joint processing (merging of the information and splitting of the actions using the joint information) of the acquired signals. These analysis and processing means 9 can operate on the acquired signals in real time or in a time-shifted manner.

Moreover, with analysis and processing means 9 an artificial intelligence application is associated (artificial intelligence methods and tools are typically "machine leaning", ANN, SVM,HMM etc.). As an artificial intelligence application an expert system 10 is preferably used, for example, that is a computer system capable of reproducing the performances of one or more people experienced in a certain field of activity, comprising an inferential motor by which, to the data coming from the analysis and processing means 9, deductive rules suitably stored in a database 11 are applied. By the expression "inferential motor" a standard or proprietary application is understood which implements an algorithm capable of drawing logical conclusions based on the user's actions detected through the incoming data from the acquisition sensors.

Through the inferential motor, the signals deriving from the two acquisition sections A and B are correlated applying deductive rules typical of the methods and tools of artificial intelligence (such as machine learning, ANN, SVM, HMM and so on).

According to the invention, processing unit 6 furthermore has an output connected to a user interface 12, by which the user obtains the classification of the features of the muscle activity detected, acquired and extracted by expert system 10. User interface 12 shows the user the results of the processing, in a graphic manner (for example on a monitor), audio manner (for example through speakers) or tactile manner; such results expressed through user interface 12 depend on the type of application to which the system is dedicated (rehabilitation, games, sports).

The contents of database 11 from which expert system 10 obtains information depend on the reference context: so, for example, for an application in a gym, information on loads to be lifted is provided, for a videogame application, information on playing controls is provided and so on. The database typically contains the user's information such as weight, fat mass, height, age, gender, which are used within the expert system, to draw the logical conclusions on the action performed by the user; database 11 furthermore contains the results of the previous actions, so that the expert system may carry out and supply the user with comparisons between the different actions or between same actions performed at different times (i.e. to show the improvement and progression in the exercises).

In database 11 the information concerning the users' physiological parameters (body mass index, fat mass index, height, weight, anthropometric measurements, etc.) and the information on the actions thereof can be stored for example.

In more general terms, it is understood that the measurement and the acquisition of the signals is carried out in a parametric manner, also based on the choice of sensors. In other words, both the sensor accuracy (and consequently also the cost) and the accuracy in supplying the measurement is chosen and/or adjusted depending on the type of application. As an example, a system developed for a professional sportsperson and that for an amateur sportsperson may share the same inferential motor and the same rules and models for signal processing, but they provide different choices in terms of hardware and software equipment. As a result, there is a difference in terms of processing time, accuracy and amount of information released by the system according to the developed application.

According to a further embodiment, the system can operate also by integrating the information coming from electromyographic sensor 3 and from video sensor 5 with other information coming from one or more further sensors which detect significant quantities of the dynamic movement of the muscle apparatus of interest. Such a further sensor can be an accelerometer, a gyroscope, an encoder, a movement sensor, a position sensor, a speed sensor, a proximity sensor, a contact sensor, at least a further display sensor, and so on. Similarly, the system can integrate the information coming from sections A and B with that coming from one or more further sensors which detect significant quantities of the muscle effort exerted by the user.

In particular, a sensor which has proved extremely advantageous to be employed in the system according to the invention is a GSR (galvanic skin resistance) sensor: through the measurement by this sensor, the system obtains a measurement of the skin impedance variation (for example due to perspiration); using this measure, the system is able to adjust to the user's specific conditions, so as to supply normalised and reliable output data; in particular, the detected impedance value is used by the system to vary in real time the gain of the EMG sensors and as further information to be entered in the inferential motor.

Other sensors to be used for any channel of the electromyographic sensor 3 are a force sensor (load cell) and the like, or sensors for detecting quantities of other physiological parameters of the user (such as heartbeat sensors, sensors for blood pressure, temperature, dilation of the pupil, ...).

In a particularly preferred embodiment, the system furthermore provides to employ sensors of a type and in a number sufficient to supply information characteristic of the user's emotional state (stress level, excitement, calmness, etc.), through special methods, known per se as "affective computing". Thereby the system is also able to measure the user's emotional state during use; this detection is important in various application sectors, for example during a game session, the system (and possibly also the game) can record the emotional data in the database and/or vary the game reaction or the administration of the exercises according to the detected emotional state.

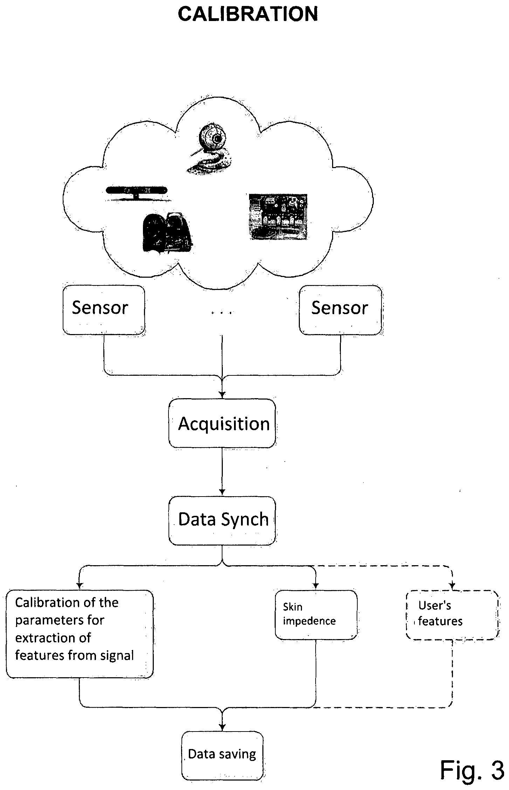

Fig. 3 shows a flow chart of the conceptual operation of the system. The apparatus part (hardware) just described is caused to operate according to a first calibration phase, to calibrate the acquired information and to possibly train the expert system for the special context in which it is to operate, a second step of regimen acquisition, with a subsequent step of data processing and output of the resulting data. With such resulting data it is possible to preferably act with feedback on the acquisition step.

In the following the various steps of the process according to the present disclosure are described in detail.

As a preliminary matter, electrodes 2 which serve to pick up the electric signal issued by the muscles during the operation thereof are positioned on the user's body part in question. Electrodes 2, which in the case shown are contained in a garment, might also be applied directly onto the user's skin without the aid of a garment. Electrodes 2 are connected to the electromyographic acquisition card 3, which in turn may be contained in a garment. The system may comprise multiple acquisition cards and multiple electrodes. Electromyographic card 3 is connected by cable and/or wirelessly to electronic processor 6. Video sensor 5 is then positioned so that it suitably frames the user, or at least the specific muscle apparatus whereon also electrodes 2 are installed; in order to improve the video image recognition capabilities by the computer system, the application of optical markers 14 (that is, objects uniquely recognised by video sensor 5 with respect to the rest of the scene) may possibly be provided. In the case shown in fig. 1B, wherein video sensor 5 uses optical markers 14, said markers must be positioned on the user's body or inserted in garments in preset positions, so as to allow video sensor 5 to acquire the user's complex movements through the tracking of known points (coinciding with optical markers 14) integral with the body parts. In the case of a video sensor 5 which employs no markers, the movement of the user's body parts is recognised and reconstructed by the computer system through suitable optical recognition algorithms (for example the ones employed in some game consoles, such as kinect® by Microsoft Corporation or the controller Playstation Move® by Sony Computer Entertainment Inc.).

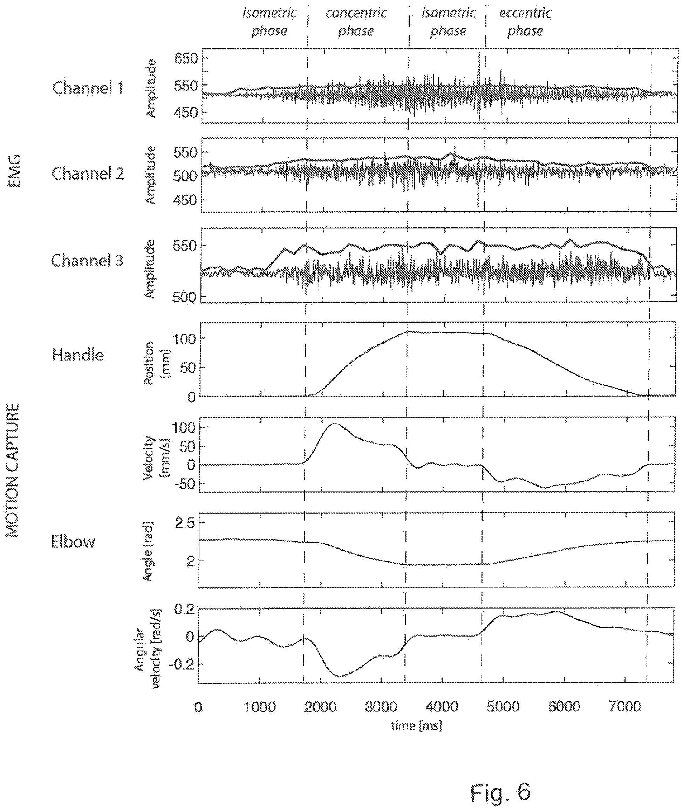

Through the two acquisition sections A and B, signals (directly digital, or analogical ones subsequently converted into digital ones) both of the movements of the user's body parts (for example of either arm) and of the electric impulses generated in the muscles on which electrodes 2 are installed are acquired. Fig. 6 shows, in an exemplifying manner, seven diagrams of signals acquired over time, the first three referring to three different channels of the electromyographic sensors and the subsequent four referring to the video sensors. In particular, from fig. 6 one can understand that from the video sensors a dynamic image of the movement can be obtained, from which it is then possible to extract multiple parameters: for example from each channel (on one side the hand and on the other side the elbow) the position and velocity in time information (data) are extracted.

In particular, in the first three quadrants the amplitude of the electric signal acquired by three respective electrode channels is shown; in the fourth and fifth quadrant the position signal and the velocity signal, respectively, of the hand along a reference axis in time are shown; in the sixth and seventh quadrant the angular position signal and the angular velocity signal, respectively, of the elbow around a reference axis in time are shown.

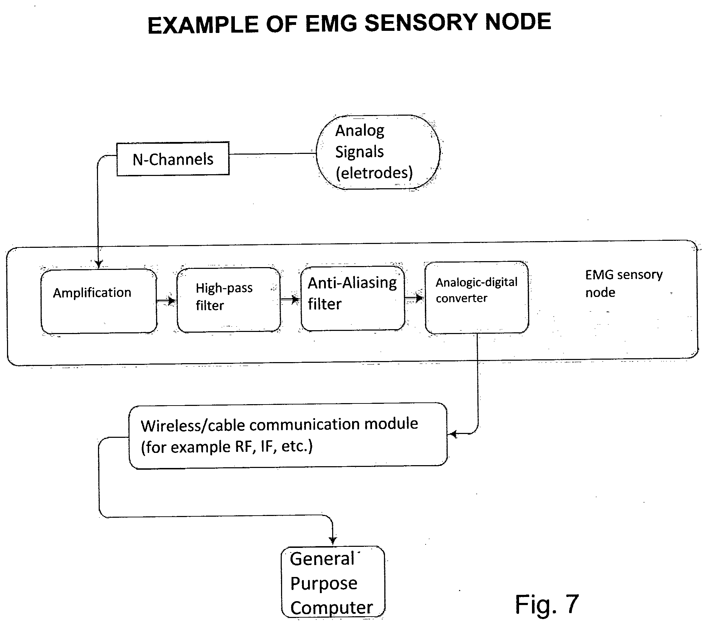

Fig. 7 shows an exemplifying acquisition process performed in the electromyographic acquisition section.

One or more electrodes acquire a signal of the electric activity in the muscles in an analogic manner, preferably on multiple channels (N). Inside the EMG card, the signals are duly amplified with a certain gain and then a high-pass filter and an anti-aliasing filter are applied. Before transferring the signal to the processing unit, the signal is converted into digital form (A/D converter) and sent by cable or wirelessly to a communication module, through which it is sent to the processing unit, which may also be in the form of a general-purpose personal computer.

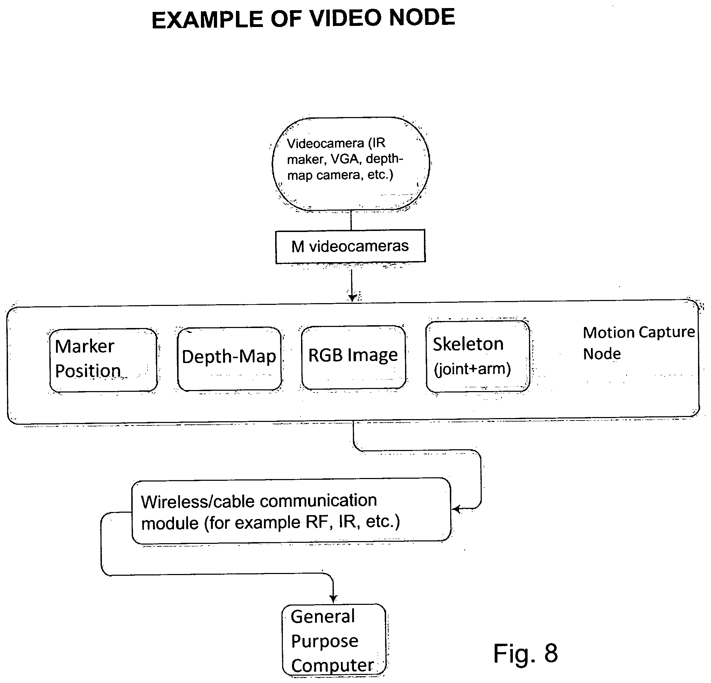

Fig. 8 shows an exemplifying acquisition process performed in the video acquisition section.

One or more digital video devices (webcam, video camera, multisensor camera, ...) frame a part or the entire body of the individual, acquiring data useful for building more complex information - through algorithms which are known per se - such as the position of any markers and/or a depth-map and/or an rgb image and/or the skeleton of that portion (for example arm+elbow). This complex information is sent to a communication module which transfers it to the processing unit.

During system operation other signals coming from additional sensors can also be acquired, such as those mentioned above.

The signals are sampled in real time in a preset time frame depending on the context. The reference time frame hence defines a reference clock by which the time synchronisation of the various signals acquired is obtained.

Thereby it is possible, along time, to obtain the sensor readings synchronised following a single reference time signal supplied by the signal synchroniser.

The information from the sensors of the two acquisition sections A and B, and possibly from the other sensors (but at least from the GSR sensor), are subsequently merged, in the processing unit for the subsequent extraction of information concerning the user's actions, due to the fact that all the acquisitions by any sensor refer - as stated - to a single time clock (timestamp). It is hence possible to determine if, for example, a user is making an isometric effort (i.e. an action even without specific movement) or even if a plurality of movements performed with the same velocity and acceleration components require different muscle efforts, as for example in the case of a lifting exercise, which is performed with the same velocity and accelerations but with different loads (=weights).

As shown in fig. 3, in the first calibration phase, the data acquisition and synchronisation operation occurs as indicated above, but having previously prompted the user to enter some of his/her physiological parameters (height, weight, gender, age, etc.) and to perform some preset activities in order to initialise some work parameters. In particular, the user is prompted to perform preset movements and make preset efforts to tune the system acquisition scales, removing noise-caused offsets, due for example to electrode degradation or to the incorrect positioning of the same (in this last case the user will be warned of the incorrect positioning). In this phase, therefore, the system can analyse a user's action of which it semantically knows the meaning of in the reference context (for example, lifting a weight during an exercise at the gym, or performing a rightward shifting action of the arm for the simulation of a control and so on). This activity allows to obtain a tuning of the signals, which are representative of standard actions known by the system.

In order to analyse the action, analysis and processing means 9 extract from the detected raw data all the information necessary to assign a correct meaning to the action analysed in the context of reference. For each signal thus sampled, analysis and processing means 9 extract a piece of data or data set which represents relevant characterising information of the signal in the time frame. In order to obtain the characterising information, operations or extractions of the signal values are performed; for example, the characterising information may be the mean, the absolute value, the position derivative, and so on. In the calibration phase a significant role is played by the acquisition of the skin impedance, through the GSR sensor described above. In particular, in the system an impedance sensor is provided for measuring skin impedance.

Such impedance sensor is connected to computer processor 6 and may be integrated or not in the electromyographic acquisition section and allows to measure the skin impedance through at least two detection electrodes.

Such impedance measure is advantageously used to adjust in real time the gain of each channel of the electromyographic sensor: in particular the gain is changed in a way directly proportional to the detected impedance value so as to calibrate the corresponding scales; for example the acquisition of electromyographic data performed with perspiring skin and - the action (effort, velocity etc.) being equal - performed with dry skin, would supply per se a very different reading and would hence not render the two exercices comparable, which exercises are instead equal from a dynamic and kinematic point of view. The detection of the skin impedance allows to recognise the two different conditions and hence to correct the acquired data to normalise and make them comparable in the different skin conditions. Moreover, the skin impedance signal supplies a measure of perspiration and is hence a supporting value in the measuring of the user's fatigue during the actions.

This characterising information, extracted by analysis and processing means 9, is then saved at the end of the calibration phase to be used by expert system 10 to then classify, based on the context (information contained in the database), the action performed in the runtime phase.

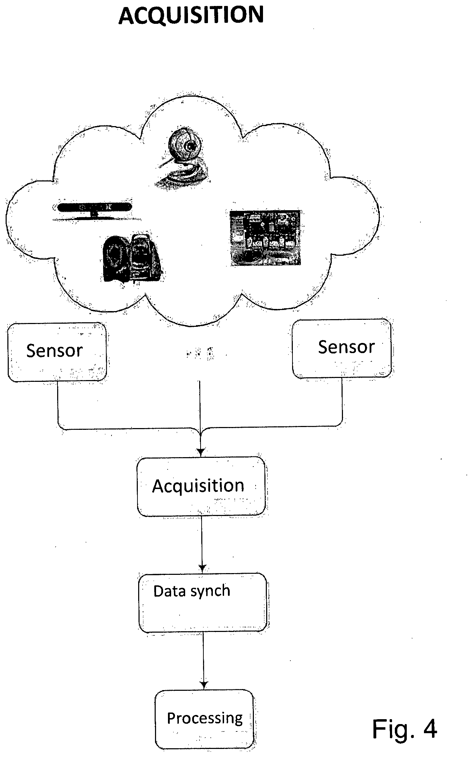

Fig. 4 shows a flow chart of the runtime acquisition step. The first part, comprising sensor detection, signal acquisition and data synchronisation, occurs as in the calibration step. The subsequent processing part relies on the data saved during the calibration phase.

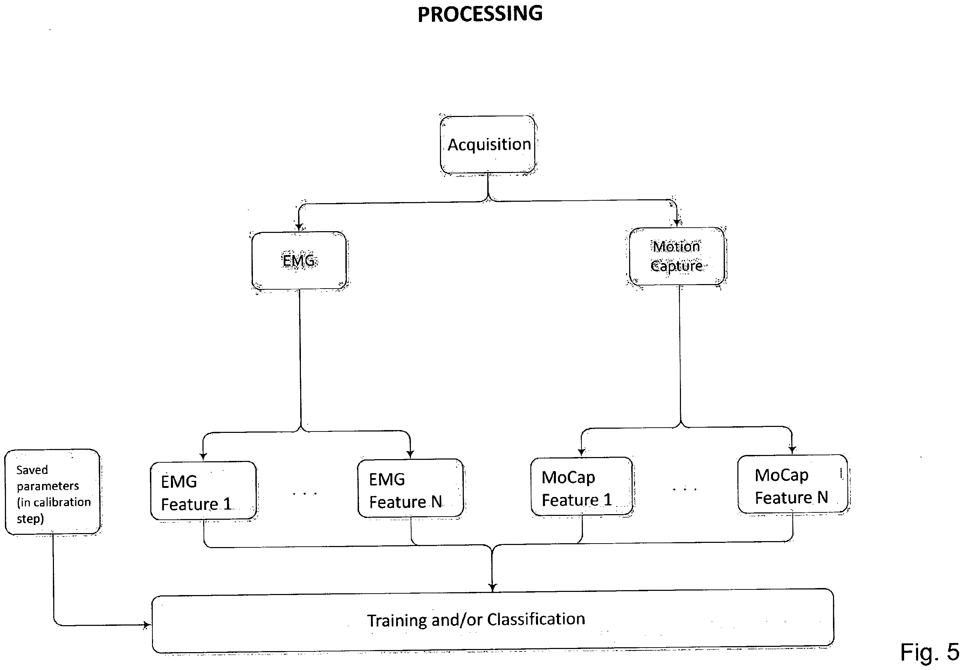

As better shown in fig. 5, in the runtime processing step, from the acquired signals the characterising information is extracted (feature$_1$, ..., feature$_N$) and then, based on the parameters saved during the calibration phase, a signal classification is performed to then be able to supply an output (control of an apparatus, control of a representation of an object on a display, processed statistic data, ...) suited to the specific application.

In particular the acquired synchronised signals are analysed and processed, then the data deriving from the analysis and processing are transmitted to expert system 10 which performs the classification of the characterising information of the muscle activity. In particular, the synchronised signals coming from the different sensors are filtered and cleaned from possible noise. Subsequently, from such raw information, various more complex features, such as average, frequency, positions, velocity, accelerations, jerks of the body components are derived.

In table 1 reported here below some of the features derivable from the EMG signal are listed.

Table 1

| FEATURE | VALUE | COMMENT |
|---|---|---|
| FREQUENCY DOMAIN | | |
| *Histogram of EMG* (HEMG) | Hist(sEMG) | It divides the elements in sEMG signal into b equally spaced segments and returns the number of elements in each segment. b has to be decided heuristically. Together with WAMP it has showed the best result for recognition in a noisy environment. |

(continued)

| FEATURE | VALUE | COMMENT |
|---|---|---|
| FREQUENCY DOMAIN | | |
| *Integral of absolute value (IAV)* | $$IAV = \sum_{i=1}^{N} |x_i|$$ | It is calculated as the summation of the absolute values of the sEMG signal amplitude. Generally, IEMG is used as an onset index to detect the muscle activity that used to oncoming the control command of assistive control device. It is related to the sEMG signal sequence firing point. |
| *Mean Absolute Value (MAV)* | $$MAV = \frac{1}{N} \sum_{i=1}^{N} |x_i|$$ | It is an easy way for the detection of muscle contraction levels and it is a popular feature used in myoelectric control application. |
| *Mean Absolute Value Slope (MAVSLP)* | $MAVSLP_i = MAV_{i+1} - MAV_i$ | Mean Absolute Value Slope (MAVSLP) is a modified version of MAV. The differences between the MAVs of adjacent segments are determined. |
| *Modified Mean Absolute Value 1 (MMAV1)* | $$MMAV1 = \frac{1}{N} \sum_{i=1}^{N} |x_i| w_n$$ $$w_n = \begin{cases} 1, & 0.25N \le n \le 0.75N \\ 0.5, & otherwise \end{cases}$$ | It is an extension of MAV using weighting window function $W_n$. |
| *Modified Mean Absolute Value 2 (MMAV2)* | $$MMAV2 = \frac{1}{N} \sum_{i=1}^{N} |x_i| w_n$$ $$w_n = \begin{cases} 1, & 0.25N \le n \le 0.75N \\ 4n/N, & 0.25N > n \\ 4(n-N)/N, & 0.75N < n \end{cases}$$ | It is similar to MMAV1. However, the smooth window is improved in this method using continuous weighting window function $W_n$. |
| *Root mean square (RMS)* | $$RMS = \sqrt{\frac{1}{N} \sum_{i=1}^{N} x_i^2}$$ | It represents the muscle activity for each channel. The processing of the MAV feature is equal to or better in theory and experiment than RMS processing [14]. Furthermore, the measured index of power property that remained in the RMS feature is more advantage than in the MAV feature. |

(continued)

| FEATURE | VALUE | COMMENT |
|---|---|---|
| FREQUENCY DOMAIN | | |
| *Slope Sign Change* (SSC) | $$ZC = \sum_{i=1}^{N-1} f[(x_i - x_{i-1})(x_i - x_{i+1})]$$ $$f(x) = \begin{cases} 1, & x > threshold \\ 0, & otherwise \end{cases}$$ | It is another method to represent the frequency information of the sEMG signal. The number of changes between positive and negative slope among three consecutive segments are performed with the threshold function to avoid the interference in sEMG signal.in sEMG signal. |
| *Simple Square Integral* (SSI) | $$SSI = \sum_{i=1}^{N} |x_i|^2$$ | It is the energy of the sEMG signal. |
| *Variance* (VAR) | $$VAR = \frac{1}{N-1} \sum_{i=1}^{N} x_i^2$$ | The power of the sEMG signal (written in this manner because mean is almost 0). |
| *Willison Amplitude* (WAMP) | $$WAMP = \sum_{i=1}^{N} f(|x_i - x_{i+1}|)$$ $$f(x) = \begin{cases} 1, & x > threshold \\ 0, & otherwise \end{cases}$$ | Willison Amplitude is the number of times that the difference between sEMG signal amplitude between two adjacent segments exceeds a predefined threshold, to reduce noise effects. This feature is an indicator of firing of motor unit action potentials (MUAP) and therefore an indicator of muscle contraction level. |
| *Waveform length* (WL) | $$WL = \sum_{i=1}^{N-1} |x_{i+1} - x_i|$$ | It is the cumulative length of the waveform over the time segment. WL is related to the waveform amplitude, frequency and time. |
| *Zero crossing* (ZC) | $$ZC = \sum_{i=1}^{N} [sgn(x_i x_{i+1}) \cap |x_n - x_{n+1}| \geq th$$ $$sgn(x) = \begin{cases} 1, & x > threshold \\ 0, & otherwise \end{cases}$$ | It counts the number of times the signal crosses the zero amplitude axis. This feature provides an approximate estimate of frequency domain properties. The threshold condition is used to abstain from the background noise. |
| FREQUENCY DOMAIN | | |
| *Autoregressive* (AR) | *AR = AR(sEMG)* | The autoregressive model described each sample of sEMG signal as a linear combination of previous samples plus a white noise error term. AR coefficients are used as features. Various algorithms used to calculate them. Good results have been obtained with 4th- order coefficients. |

(continued)

| FREQUENCY DOMAIN | | |
|---|---|---|
| **Median Frequency (MDF)** | $$\sum_{i=1}^{MDF} P_i = \sum_{i=MDF}^{M} P_i = \frac{1}{2}\sum_{i=1}^{M} P_i$$ $A_i$ is the sEMG amplitude spectrum at frequency bin i | The frequency at which the spectrum is divided into two regions with equal amplitude. |
| **Modified Median Frequency (MMDF)** | $$\sum_{i=1}^{MMDF} A_i = \sum_{i=MMDF}^{M} A_i = \frac{1}{2}\sum_{i=1}^{M} A_i$$ $A_i$ is the sEMG amplitude spectrum at frequency bin i | It is the amplitude frequency at which the spectrum is divided into two regions with equal amplitude. The outline of amplitude spectrum and power spectrum is similar but the amplitude value of amplitude spectrum is larger than the amplitude value of power spectrum. Moreover, the variation of amplitude spectrum is less than the power spectrum. For that reason, variation of MMNF and MMDF is also less than traditional MNF and MDF. |
| **Modified Mean Frequency (MMNF)** | $$MMNF = \left.\sum_{i=1}^{M} f_i A_i \middle/ \sum_{i=1}^{M} A_i\right.$$ $f_i$ is the frequency of spectrum at frequency bin j. | It is the average amplitude spectrum. |
| **Mean Frequency (MNF)** | $$MNF = \left.\sum_{i=1}^{M} f_i P_i \middle/ \sum_{i=1}^{M} P_i\right.$$ $f_i$ is the frequency of spectrum at frequency bin j. | I is the average frequency. |
| TIME-FREQUENCY DOMAIN | | |
| **Singular Value Decomposition of CWC (SVD)** | *SVD = SVD(CWC)* | The Singular Value Decomposition of the Continuous Wavelet Transform coefficients. It finds n features (where n is the number of amplitudes taken into consideration), n has to be defined heuristically. Very good results have been obtained using this feature for gesture classification. |
| **Weighted sum of absolute value** | | No physical significance, but it has proven to be useful for signal classification. |
| **of CWC (SA)** | $$SA = W\sum_{i=1}^{N} |CWC(\tau,\sigma)|$$ $$W = \frac{1}{N}\sum_{i=1}^{N} |x_i|$$ | Relatively fast to calculate. |

(continued)

| TIME-FREQUENCY DOMAIN | | |
|---|---|---|
| *Weighted standard deviation of CWC* (SD) | $$SD = W\left(\sqrt{\frac{1}{N-1}\sum_{i=1}^{N}\left(CWC(\tau,\sigma) - \overline{CWC(\tau,\sigma)}^2\right)}\right)$$ $$\overline{CWC(\tau,\sigma)} = \frac{1}{N}\sum_{i=1}^{N} CWC(\tau,\sigma)$$ | |
| *Weighted variance of CWC* (VR) | $VR = SD^2$ | |
| *Weighted fourth central moment of CWC* (CM) | $CM = ...$ | |
| *Weighted skewness of CWC-SS* (SK) | $SK = ...$ | |
| *Weighted kurtosis of CWC-SS* (KU) | $KU = ...$ | |

The kinematic features are entered in the expert system always in a correlated manner, due to the common clock, to the information supplied by the EMG and GSR signals and in any case by all other optional sensors provided in the system. The ways to extract information from the EMG signal are known per se: as an example, see what is described in Md. R. Ahsan, Muhammad I. Ibrahimy, Othman O. Khalifa, "EMG Signal Classification for Human Computer Interaction: A Review", European Journal of Scientific Research Vol.33 No.3 (2009), pp.480-501.

The same data deriving from the analysis and processing may be used for the training of expert system 10.

By this acquisition and analysis system and method the level of contraction of the affected muscle or muscle districts can be described, the fatigue of the muscles or of the muscle districts, the synchronisation of muscle activations, the movement synchronisation, the action kinematics (positions, velocities, accelerations) may be described. It is hence possible to divide each action into sub-actions, mutually differentiating them according to the type of the same, for example differentiating a movement between the dynamics and the statics, both from the point of view of the movement (when present), and of that concerning muscle activation. It is hence possible to refer muscle activation to the different types of subaction for recognising activated muscle groups, recognising the actions, detecting the level of fatigue during an action, distinguishing the different types of contraction, enabling the user to improve the execution of a given exercise/gesture, detecting the anomalies with respect to the correct execution of the exercise, monitoring training/rehabilitation progress.

Among other things, the disclosure allows to implement a new human-machine (computer) interaction, for example for videogames (the control is expressed in terms of movement + muscle effort).

Output information, as said, may be supplied to a user in different ways on different hardware/software platforms, mobile or fixed devices (for example smartPhone, tablet, pc desktop etc.). This information can furthermore be supplied through video and/or audio and/or tactile devices.

The system output data may advantageously be used also for a feedback check, typically for a self-adjustment of the system configuration. As a matter of fact, the detection of acquisition section data which should prove inconsistent with respect to the ones expected based on the information coming from the other section may trigger the action of self-adjustment means.

Similarly, self-adjustment may intervene automatically from an inconsistency signal deriving from the comparison of a combination of the electromyographic signal and of the video signal with a previous-knowledge basic archive.

The apparatus is hence capable of adapting the physical configuration thereof to improve acquisition quality based on the information extracted by the above sensors. In particular, based on the information extracted from the video and/or electromyographic signals, the system is capable - based on previous knowledge (knowledge base) of the subject's movement - of identifying processing anomalies of the signal.

In case a consistent electromyographic signal and an inconsistent video signal are obtained, the system may be able to act and change (of course if the specific configuration allows it) the operation modes for video acquisition, for example the level of zoom and/or panoramic (PAN) and/or inclination (TILT) of the video sensor. The video system is thus ordered to focus the attention on the parts detected in an abnormal way based on correct electromyographic acquisition.

The apparatus is hence capable of solving the anomaly and to configure itself correctly, so as to allow a robust and reliable acquisition also of those parts which might possibly be neglected with actuation.

Vice versa, in case a video signal is obtained which highlights a body movement which should result - based on the information stored in the database and interpreted with the inferential motor of expert motor 10 - from the activation of one or more muscle groups, but the electromyographic signal should not detect such activation, the apparatus according to the invention is able to change in real time, for each channel of the electromyographic sensor, the gain thereof, in order to obtain a correct display of the electromyographic signal.

As seen above, moreover, the gain of the electromyographic sensors is changed according to the impedance value detected by the corresponding skin sensor.

The system allows to reconstruct in 3D the human figure (known as skeleton in the jargon) using the video signal. The skeleton allows to compute the subject's movement in 3D space (for example velocity, limb acceleration, movement trajectory, etc.).

In this context, by the term skeleton a representation of the user's body and of the interaction thereof with the system, in qualitative and quantitative terms, is meant to be designated: typically, the skeleton is a representation which includes, in addition to a geometric image of the user's body, also the relative movements, efforts, fatigue conditions, errors and other user conditions. The skeleton representation may be supplied with an interface which may be graphic (video), audio (speaker) or tactile.

The extraction of the skeleton from the video signal is possible through algorithms known in the literature. Any abnormal movements are identified using the above skeleton and the EMG signals correlated to such movement: in the light of the previous training of the expert system and the time sequence of the movements being known, the system is automatically capable of identifying movements which are not compatible with the knowledge base. For example, the expert system identifies the kinematics and the muscle effort and assesses that they are not correct: it is hence identified whether the movement is incorrect and to what extent/how it is incorrect (for example if a movement is performed in advance or delayed with respect to another, hence not providing the correct coordination). Alternatively, the expert system may determine that, with respect to the type of set training/game, the type of the user's movement/exercise (i.e. the weight, the number of repetitions, the fatigue, the stress if the optional *sensors for affective computing* are provided) is not correct.

As mentioned above, the disclosed system may advantageously be used in various fields, such as: sports (professional; for gyms, integrated in the equipment; home/hobby, for home training through suitable software installed on personal computer/console), rehabilitation (hospital/physiotherapy centre; home, through suitable software installed on personal computer/console), play (games on computer and console).

In gyms or in the physiotherapy field, the system is capable of comprising the kinematics and the dynamics of a user's actions and of guiding said user towards the correct execution of the sport gesture, correcting the position thereof, execution time and the correct use of the body, in order to improve both the technique and the user's wellbeing or to guide the user independently, hence without the help of an expert (for example a physiotherapist), in the correct execution of post-traumatic rehabilitation exercises. The system, at the end of the processing, may also supply a performance level mark to the user, so that the user may easily and intuitively compare it against a desired performance model.

In a video-game context, with the disclosed system it is possible to define output controls which take into account, in addition to the user's movements, also the level of force, the level of muscle fatigue, movement synchronisation and muscle activation velocity. Thereby it is possible to recreate new ways of interaction, not present on the market so far, significantly improving the users' level of involvement and allowing the creation of a new videogame category.

In all these contexts it is feasible to share some outcomes of the system processing in social networks.

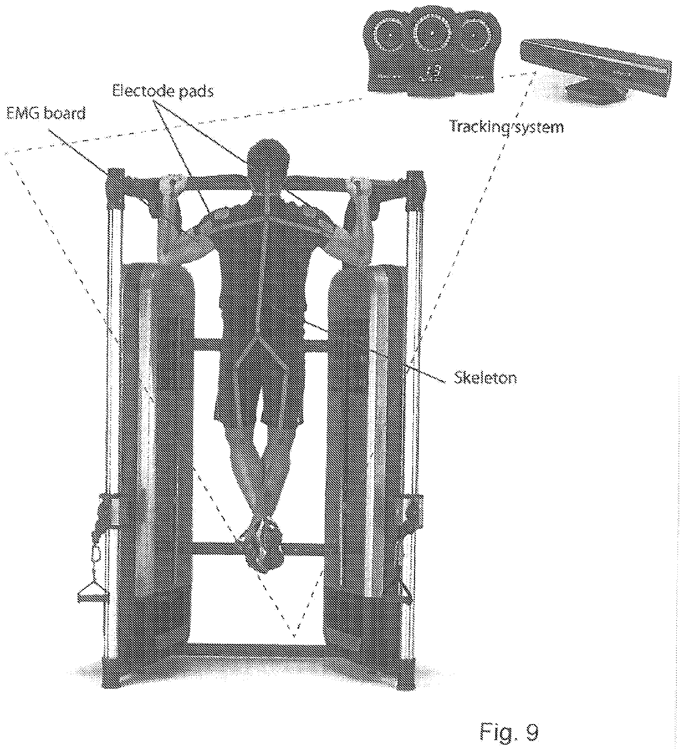

Fig. 9 shows a possible application of the disclosed system to a piece of equipment for physical exercise and sports training.

While a user performs a physical exercise at a muscle training machine, a video device acquires the image of the moving skeleton; at the same time a series of electrodes embedded in the user's training outfit acquires a series of signals on multiple respective channels, for example in correspondence of the arm muscles, the back muscles and the shoulder muscles. The treatment of the data with the system of the invention allows to supply as output an indication of the congruence of the movements with the electromyographic signals, and vice versa, based on the expected model. Should the system detect incongruence or discrepancy in the correlation between the data received by the two acquisition sections and the expected one, it issues a correction indication (for example in the form of a visual warning on a display) which leads the user to change posture or effort in a way consistent with the reaching of the desired and pre-stored model or the model built in the calibrating step in the system database.

However, it is understood that the invention must not be considered limited to the particular arrangements illustrated above, which are only exemplifying embodiments, but that different variants are possible without departing from the scope of protection of the invention, as defined by the following claims.

In particular, many of the elements exemplifyingly described in the present invention may be replaced by technically equivalent elements. In practice the materials used, as well as the size, can be any one, depending on the requirements and the state of the art.

Although no specific indications are provided on the features of the processing unit in which the analysis and processing means provided with the expert system with inferential motor are included, it may be a personal computer or a mobile device with calculating capabilities, locally arranged (with respect to the two data acquisition sections), or a remotely located server, through which calculating capacity is dispensed to a series of similar networked systems.

**Claims**

1. System for the acquisition and analysis of an individual's muscle activity, comprising
   at least an electromyographic acquisition section (A) apt to acquire through respective sensors at least first electric signals of an individual's muscle group,
   a garment (1) belonging to said acquisition section (A), being provided with electrodes (2) and a surface electromyographic acquisition card EMG (3) with one or more channels for each electrode (2) or pair of electrodes, capable of acquiring, filtering and sampling the electric signal issued during a muscle contraction,
   a computer processor (6) furthermore having analysis and processing means (9) for the analysis and processing of the acquired signals,
   a user interface (12) through which to supply an output processed by said computer processor (6),
   a communication interface with said electromyographic section (A), and
   at least one detection sensor of the individual's skin impedance is further provided, apt to determine an impedance value
   **characterised in that**
   said computer processor further comprises a database (11) of deductive rules,
   said processing and analysis means (9) are provided with an expert system (10) in the shape of a computer system capable of reproducing the performances of one or more people experienced in a certain field of activity, comprising an inferential motor by which, to the data coming from the analysis and processing means (9), deductive rules suitably stored in said database (11) are applied, and **in that**
   the system provides to use such a impedance value as correction parameter of the gain of said sensors of the electromyographic acquisition section (A).

2. Method for the acquisition and analysis of an individual's muscle activity, comprising the phases of employing a system as claimed in claim 1 to
   acquire at least first electric signals of an individual's muscle group through an electromyographic acquisition section (A),
   transmitting said first signals to a computer processor (6) provided with analysis and processing means (9) for the analysis and processing of the acquired signals and an expert system (10) employing an inferential motor through which deductive rules, provided in a database (11) connected to said computer processor (6), are applied to said signals,
   using an impedance value, determined by a detection sensor of the individual's skin impedance, as correction parameter of the gain of said sensors of the electromyographic acquisition section (A), and
   issuing the outcome of said correlation to a user interface (12).

3. Method as claimed in claim 2, wherein a detection step of the impedance value of the user's skin is provided, in correspondence of sensors (2) of said electromyographic acquisition section (A), and a subsequent adjustment of the gain of said sensors (2) in a proportional manner to said impedance value.

4. Method as claimed in claim 2 or 3, wherein said expert system (10) is a computer system apt to reproduce the performances of one or more people experienced in a certain field of activity, said database (11) further containing the result of previous actions, so that the expert system (10) may carry out and supply the user with comparison between the different actions or between same actions performed at different times.

5. Method as claimed in claims 2, 3 or 4, wherein said user interface (12) supplies the user with a classification of features of muscle activity detected, acquired and extracted by said expert system (10), by showing said features in a graphic manner, audio manner or tactile manner.

6.  Method as claimed in any one of preceding claims 2 to 5, wherein a preliminary training step of said expert system (10) is provided, wherein said at least first signals are acquired in correspondence of known muscle activities performed by the user, so as to build a comparison model stored in said database (11).

7.  Method as claimed in any one of preceding claims 2 to 6, wherein the system comprises the kinematics and the dynamics of a user's actions and guides a user towards the correct execution of the sport gesture, execution time and the correct use of the body, while supplying, at the end of the processing, a performance level mark to the user.


**Patentansprüche**

1.  System zur Erfassung und Analyse der Muskelaktivität eines Individuums, umfassend

    zumindest einen elektromyografischen Erfassungsabschnitt (A), der geeignet ist, durch jeweilige Sensoren zumindest erste elektrische Signale der Muskelgruppe eines Individuums zu erfassen;

    ein mit dem Erfassungsabschnitt (A) verbundenes Kleidungsstück (1), das mit Elektroden (2) und einer elektromyografischen Oberflächenerfassungskarte EMG (3) mit einem oder mehreren Kanälen für jede Elektrode (2) oder Paare von Elektroden, die in der Lage sind, das bei einer Muskelkontraktion ausgegebene elektrische Signal zu erfassen, zu filtern und zu verarbeiten, versehen ist;

    ein Computerprozessor (6), der weiterhin Analyse- und Verarbeitungsmittel (9) zur Analyse und Verarbeitung der erfassten Signale aufweist;

    eine Benutzerschnittstelle (12), durch die eine vom Computerprozessor (6) verarbeitete Ausgabe bereitstellbar ist;

    eine Kommunikationsschnittstelle mit dem genannten elektromyografischen Abschnitt (A); und

    wobei mindestens ein Erfassungssensor der Impedanz der Haut des Individuums ferner vorgesehen ist, um einen Impedanzwert zu bestimmen;

    **dadurch gekennzeichnet, dass**

    der Computerprozessor ferner eine Datenbank (11) aus deduktiven Regeln umfasst;

    die Verarbeitungs- und Analysemittel (9) mit einem Expertensystem (10) in Form eines Computersystems versehen sind, das in der Lage ist, die Leistungen einer oder mehrerer Personen zu reproduzieren, die in einem bestimmten Tätigkeitsfeld erfahren wurden, umfassend eine Inferenzmaschine, durch die zu den aus den Analyse- und Verarbeitungsmittel (9) stammenden Daten, deduktive Regeln angewendet werden, die zweckmäßig in der Datenbank (11) gespeichert sind, und dadurch, dass

    das System anbietet, einen solchen Impedanzwert als eine Korrektur der Verstärkung der Sensoren des elektromyografischen Erfassungsabschnitts (A) zu verwenden.

2.  Verfahren zur Erfassung und Analyse der Muskelaktivität eines Individuums, umfassend die Phasen des Betreibens eines Systems nach Anspruch 1, um

    zumindest erste elektrische Signale der Muskelgruppe eines Individuums durch einen elektromyografischen Erfassungsabschnitt (A) zu erfassen;

    die ersten Signale an einen Computerprozessor (6) zu übertragen, der mit Analyse- und Verarbeitungsmitteln (9) zur Analyse und Verarbeitung der erfassten Signale und mit einem Expertensystem (10), das eine Inferenzmaschine einsetzt, durch welche deduktive Regeln, die von einer mit dem Computerprozessor (6) verbundenen Datenbank (11) zur Verfügung gestellt werden, auf die Signale angewendet werden, versehen ist;

    einen durch einen Erkennungssensor der Impedanz der Haut eines Individuums bestimmten Impedanzwert als Korrekturparameter der Verstärkung der Sensoren des elektromyografischen Verfassungsabschnitts (A) zu benutzen und

    das Ergebnis dieser Korrelation zu einer Benutzerschnittstelle (12) auszugeben.

3.  Verfahren nach Anspruch 2, wobei in Übereinstimmung mit den Sensoren (2) des elektromyografischen Erfassungsabschnitts (A) ein Erfassungsschritt des Impedanzwertes der Haut des Benutzers und eine nachfolgende Einstellung der Verstärkung der Sensoren (2) proportional zu dem Impedanzwert vorgesehen ist.

4.  Verfahren nach Anspruch 2 oder 3, wobei das Expertensystem (10) ein Computersystem ist, das geeignet ist, die Leistungen einer oder mehrerer Personen zu reproduzieren, die in einem bestimmten Tätigkeitsfeld erfahren wurden, wobei die Datenbank (11) ferner das Ergebnis von vorherigen Handlungen beinhaltet, so dass das Expertensystem (10) einen Vergleich zwischen den verschiedenen Aktionen oder zwischen gleichen Aktionen, die zu verschiedenen Zeiten durchgeführt werden, durchführen kann und den Benutzer damit versorgen kann.

**5.** Verfahren nach Anspruch 2, 3 oder 4, wobei die Benutzerschnittstelle (12) den Benutzer mit einer Klassifizierung von Merkmalen der durch das Expertensystem (10) detektierten, erfassten und extrahierten Muskelaktivität versorgt, in dem die Merkmale auf grafische Weise, auf akustische Weise oder taktile Weise dargestellt werden.

**6.** Verfahren nach einem der vorhergehenden Ansprüche 2 bis 5, wobei ein vorbereitender Trainingsschritt des Expertensystems (10) vorgesehen ist, wobei die zumindest ersten Signale in Übereinstimmung mit bekannten, vom Benutzer durchgeführten Muskelaktivitäten erfasst werden, um ein in der Datenbank (11) gespeichertes Vergleichsmodell aufzubauen.

**7.** Verfahren nach einem der vorhergehenden Ansprüche 2 bis 6, wobei das System die Kinematik und die Dynamik der Aktivitäten eines Benutzers umfasst und den Benutzer zur korrekten Ausführung der Sportgeste, zur Ausführungszeit und zur korrekten Verwendung des Körpers führt, während am Ende der Verarbeitung eine Leistungsniveaumarke an den Benutzer geliefert wird.

## Revendications

**1.** Système pour l'acquisition et l'analyse de l'activité musculaire d'un individu, comprenant
au moins une section d'acquisition électromyographique (A) capable d'acquérir, par l'intermédiaire de capteurs respectifs, au moins des premiers signaux électriques d'un groupe de muscles d'un individu,
un vêtement (1) appartenant à ladite section d'acquisition (A), pourvu d'électrodes (2) et d'une carte d'acquisition électromyographique EMG (3) de surface avec un ou plusieurs canaux pour chaque électrode (2) ou paire d'électrodes, capable d'acquérir, de filtrer et d'échantillonner le signal électrique émis pendant une contraction musculaire,
un processeur d'ordinateur (6) comprenant en outre des moyens d'analyse et de traitement (9) pour l'analyse et le traitement des signaux acquis,
une interface utilisateur (12) par laquelle une sortie traitée par ledit processeur d'ordinateur (6) est fournie,
une interface de communication avec ladite section électromyographique (A), et
au moins un capteur de détection de l'impédance de la peau d'un individu est en outre prévu, capable de déterminer une valeur d'impédance,
**caractérisé en ce que**
ledit processeur d'ordinateur comprend en outre une base de données (11) de règles de déduction,
lesdits moyens de traitement et d'analyse (9) sont pourvus d'un système expert (10) sous la forme d'un système informatique capable de reproduire les performances d'une ou de plusieurs personnes expérimentées dans un certain domaine d'activité, comprenant un moteur de déduction par lequel, pour les données provenant des moyens d'analyse et de traitement (9), des règles de déduction mémorisées de manière appropriée dans ladite base de données (11) sont appliquées, et **en ce que**
le système permet d'utiliser une telle valeur d'impédance en tant que paramètre de correction du gain desdits capteurs de la section d'acquisition électromyographique (A).

**2.** Procédé pour l'acquisition et l'analyse de l'activité musculaire d'un individu, comprenant les phases d'utilisation d'un système selon la revendication 1 pour acquérir au moins des premiers signaux électriques d'un groupe de muscles d'un individu par l'intermédiaire d'une section d'acquisition électromyographique (A),
de transmission desdits premiers signaux à un processeur d'ordinateur (6) pourvu de moyens d'analyse et de traitement (9) pour l'analyse et le traitement des signaux acquis et d'un système expert (10) utilisant un moteur de déduction par lequel des règles de déduction, prévues dans une base de données (11) connectée au dit processeur d'ordinateur (6), sont appliquées auxdits signaux,
d'utilisation d'une valeur d'impédance, déterminée par un capteur de détection de l'impédance de la peau d'un individu, en tant que paramètre de correction du gain desdits capteurs de la section d'acquisition électromyographique (A), et
d'émission du résultat de ladite corrélation vers une interface utilisateur (12).

**3.** Procédé selon la revendication 2, dans lequel une étape de détection de la valeur d'impédance de la peau d'un utilisateur est prévue, en correspondance avec des capteurs (2) de ladite section d'acquisition électromyographique (A), et un ajustement subséquent du gain desdits capteurs (2) d'une manière proportionnelle à ladite valeur d'impédance est prévu.

**4.** Procédé selon la revendication 2 ou 3, dans lequel ledit système expert (10) est un système informatique capable de reproduire les performances d'une ou de plusieurs personnes expérimentées dans un certain domaine d'activité,

EP 3 069 656 B1

ladite base de données (11) contenant en outre le résultat d'actions précédentes, de sorte que le système expert (10) peut effectuer et fournir à l'utilisateur une comparaison entre les différentes actions ou entre les mêmes actions effectuées à différents instants.

5. Procédé selon la revendication 2, 3 ou 4, dans lequel ladite interface utilisateur (12) fournit à l'utilisateur une classification des caractéristiques d'activité musculaire détectées, acquises et extraites par ledit système expert (10), en présentant lesdites caractéristiques d'une manière graphique, d'une manière audio ou d'une manière tactile.

6. Procédé selon l'une quelconque des revendications 2 à 5 précédentes, dans lequel une étape d'apprentissage préliminaire dudit système expert (10) est prévue, dans lequel lesdits au moins premiers signaux sont acquis en correspondance avec des activités musculaires connues effectuées par l'utilisateur, de manière à construire un modèle de comparaison mémorisé dans ladite base de données (11).

7. Procédé selon l'une quelconque des revendications 2 à 6 précédentes, dans lequel le système comprend la cinématique et la dynamique des actions d'un utilisateur et guide un utilisateur vers l'exécution correcte du geste sportif, un temps d'exécution et l'utilisation correcte du corps, tout en fournissant, à la fin du traitement, une marque de niveau de performance à l'utilisateur.

# System overview

Fig. 1A

Fig. 1B

# Logical flow

Fig. 2

EP 3 069 656 B1

# CALIBRATION

Fig. 3

# ACQUISITION

Fig. 4

**PROCESSING**

Fig. 5

EP 3 069 656 B1

Fig. 6

# EXAMPLE OF EMG SENSORY NODE

Fig. 7

# EXAMPLE OF VIDEO NODE

Fig. 8

EP 3 069 656 B1

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010104879 A
- EP 310901 A
- WO 201095636 A
- JP 2009273551 B
- US 20124579 B
- US 20110224530 A
- WO 2005086574 A
- WO 2010065067 A
- WO 2006082565 A

**Non-patent literature cited in the description**

- **MD. R. AHSAN ; MUHAMMAD I. IBRAHIMY ; OTHMAN O. KHALIFA.** EMG Signal Classification for Human Computer Interaction: A Review. *European Journal of Scientific Research,* 2009, vol. 33 (3), 480-501